# EUROPÄISCHE PATENTSCHRIFT

(11) **EP 1 182 959 B1**
(45) Veröffentlichungstag und Bekanntmachung des Hinweises auf die Patenterteilung: **15.01.2003**
(21) Anmeldenummer: 00936806.9
(22) Anmeldetag: 25.05.2000
(51) Int. Cl.: A61B 1/05

(54) **BILDAUFNEHMERMODUL SOWIE VERFAHREN ZUM ZUSAMMENBAUEN EINES DERARTIGEN BILDAUFNEHMERMODULS**
MODULAR IMAGE RECORDING SYSTEM AND METHOD OF ASSEMBLING SUCH A MODULAR IMAGE RECORDING SYSTEM
MODULE DE PRISE D'IMAGE ET PROCEDE DE REALISATION D'UN TEL MODULE DE PRISE D'IMAGE

(30) Priorität: 27.05.1999 DE 19924189
(43) Veröffentlichungstag der Anmeldung: 06.03.2002
(73) Patentinhaber: Karl Storz GmbH & Co. KG, 78532 Tuttlingen (DE)
(72) Erfinder: IRION, Klaus, M., D-78576 Liptingen (DE); SCHWARZ, Peter, D-78532 Tuttlingen (DE); KOCHER, Mark, D-71065 Sindelfingen (DE); KEHR, Ulrich, D-78532 Tuttlingen (DE)
(74) Vertreter: Heuckeroth, Volker, Dr.
(86) Internationale Anmeldenummer: EP0004792
(87) Internationale Veröffentlichungsnummer: WO00072744

(56) Entgegenhaltungen:
- US-A- 5 454 366
- PATENT ABSTRACTS OF JAPAN vol. 017, no. 468 (C-1102), 26. August 1993 (1993-08-26) -& JP 05 115436 A (OLYMPUS OPTICAL CO LTD), 14. Mai 1993 (1993-05-14)
- PATENT ABSTRACTS OF JAPAN vol. 018, no. 520 (C-1255), 30. September 1994 (1994-09-30) -& JP 06 178757 A (OLYMPUS OPTICAL CO LTD), 28. Juni 1994 (1994-06-28)
- PATENT ABSTRACTS OF JAPAN vol. 013, no. 074 (E-717), 20. Februar 1989 (1989-02-20) -& JP 63 254869 A (MATSUSHITA ELECTRIC IND CO LTD), 21. Oktober 1988 (1988-10-21)

## Beschreibung

Die Erfindung betrifft ein Bildaufnehmermodul, insbesondere für ein Endoskop, mit einem elektronischen Bildsensor, mit einer einstückigen Leiterplatte, die mit dem Bildsensor elektrisch kontaktiert ist, und mit der weiterhin zumindest ein von der Leiterplatte wegführendes Kabel elektrisch kontaktiert ist, wobei die Leiterplatte zumindest drei Abschnitte aufweist, von denen ein erster Abschnitt und ein zweiter Abschnitt voneinander beabstandet schräg oder quer zu dem Bildsensor verlaufen und ein dritter Abschnitt zwischen dem ersten und dem zweiten Abschnitt angeordnet ist, wobei der Bildsensor an einem dem dritten Abschnitt gegenüberliegenden Ende der Leiterplatte angeordnet ist.

Ein derartiges Bildaufnehmermodul ist aus JP 05 115436 A bekannt.

Die Erfindung betrifft ferner ein Verfahren zum Zusammenbauen eines Bildaufnehmermoduls, bei dem ein elektronischer Bildsensor mit einer einstückigen Leiterplatte elektrisch kontaktiert wird und die Leiterplatte mit zumindest einem von der Leiterplatte wegführenden Kabel elektrisch kontaktiert wird.

Ein derartiges elektronisches Bildaufnehmermodul findet allgemein in der Videoaufnahmetechnik Anwendung. Neben der Verwendung in Videokameras werden solche elektronischen Bildaufnehmermodule in jüngerer Zeit in möglichst miniaturisierter Bauform insbesondere in Endoskopen für technische oder medizinische Zwecke eingesetzt. Derartige Endoskope bzw. Videoendoskope sind beispielweise aus der US 5,754,313 und der US 5,166,787 bekannt.

Ein Bildaufnehmermodul umfaßt allgemein einen elektronischen Bildsensor bzw. Bildaufnehmer, der auf ihm abgebildetes Licht in ein elektrisches Signal umwandelt. Allgemein sind solche elektronischen Bildsensoren in CCD oder CMOS-Technologie ausgeführt. Das Bildaufnehmermodul umfaßt weiterhin zumindest eine Leiterplatte bzw. Leiterplatine, auf der sich die Signalelektronik oder zumindest ein Teil der Signalelektronik für den Bildsensor befindet. Die Signalelektronik umfaßt Schaltkreise, die durch elektronische Bauelemente oder in Form von aufgedruckten Schaltungen auf der Leiterplatte realisiert sind. Des weiteren umfaßt das Bildaufnehmermodul zumindest ein von der Leiterplatte wegführendes Kabel, das fernab von dem Bildaufnehmermodul mit einer Ansteuerungs- und Signalverarbeitungselektronik verbunden ist, die die vom Bildaufnehmermodul empfangenen elektrischen Signale zur Wiedergabe des vom Bildsensor empfangenen Bildes auf einem Bildwiedergabegerät, beispielsweise einem Display oder Monitor, verarbeitet.

In der Regel ist mit der Leiterplatte jedoch nicht nur ein einziges Kabel, sondern ein System von mehreren Kabeln zum Teil in Koaxialausführung kontaktiert, die das elektrische Signalübertragungssystem zwischen dem Bildaufnehmermodul und der Ansteuerungs- und Signalverarbeitungselektronik bilden.

Der Einsatz derartiger Bildaufnehmermodule in Endoskopen ist erst durch die Miniaturisierung der Bildsensoren und der Fortschritte in der Mikrotechnologie möglich geworden. Das Bildaufnehmermodul ist in einem Endoskop in der distalen, d.h. dem Patienten zugewandten Spitze des Endoskopschafts angeordnet, wie dies in der US 5,166,787 beschrieben ist. Das Bildaufnehmermodul ersetzt dabei die bei "klassischen" Endoskopen vorgesehene Bildübertragungsoptik, die aus einer Reihenanordnung von Linsen gebildet wird. Anstatt das distalseitig empfangene Bild mittels einem optisch abbildenden Linsensystem nach proximal, d.h. zum patientenfernen Ende, zu übertragen, werden bei einem Bildaufnehmermodul die optischen Lichtsignale in elektrische Signale umgewandelt und über zumindest ein Kabel bzw. in der Regel ein Kabelsystem nach proximal übertragen. Die Verwendung von elektronischen Bildaufnehmermodulen anstelle von optisch übertragenden Linsensystemen hat gemäß der zuvor genannten US-Patentschrift den Vorteil, daß das Bildaufnehmermodul nicht starr in die distale Spitze des Endoskopschafts eingebaut werden muß, sondern dort beweglich angebracht werden kann, so daß nach dem Einführen des Endoskops in den zu inspizierenden Hohlraum das Bildaufnehmermodul nach distal aus dem Schaft herausgeschoben oder seitlich aus dem Schaft herausgeschwenkt werden kann, wodurch dann einerseits erreicht wird, daß ein größeres Gebiet durch den beweglichen Bildsensor eingesehen werden kann, und daß andererseits der Endoskopschaft freigegeben wird, so daß beispielsweise durch den Schaft Instrumente in den Hohlraum eingeführt werden können. Außerdem ist auch eine teleskopartige Verlängerung des Endoskops denkbar.

Bei Endoskopen besteht stets die Forderung nach einem möglichst geringen Querschnitt der Außenkontur des Schafts. Dementsprechend müssen die verwendeten Bildaufnehmermodule, um in einem derartigen Schaft Platz zu finden, einen möglichst geringen Außenquerschnitt aufweisen. Beispielsweise beträgt der Schaftdurchmesser bei einem Videoendoskop für medizinische Zwecke nur wenige Millimeter (<10mm). Dies bedeutet, daß die Abmessungen des miniaturisierten Bildaufnehmermoduls so gering wie nur möglich ausgeführt sein sollten (möglichst ≤6mm). Während derzeit verfügbare Bildsensoren in sogenannter "gehäuseloser" Ausführung mit biegbaren Anschlußfingern diesen Anforderungen zunehmend genügen, besteht das Problem einer möglichst klein bauenden Ausführung des Gesamtaufbaus des Bildaufnehmermoduls weiter.

Das aus der US 5,754,313 bekannte Bildaufnehmermodul weist zwei separate, d.h. nicht einstückige Leiterplatten zur Kontaktierung des Bildsensors auf, wobei die beiden Leiterplatten in üblicher Weise elektronische Miniaturbauteile aufnehmen und zur Kontaktierung des Kabelsystems dienen. Die Leiterplatten verlaufen dabei parallel zueinander und in etwa rechtwinklig zur Bildaufnahmefläche des Bildsensors. Da die Signalelektronik auf den beiden Leiterplatten nicht unabhängig funktionieren kann, muß eine elektrische Verbindung, beispielsweise in Form von Leitungen oder einer Verbindungsleiterplatte, zusätzlich integriert werden, wodurch der Montageaufwand des Bildaufnehmermoduls erhöht ist. Außerdem erweist sich die Kontaktierung der Kabel und Elektronikkomponenten zwischen den Platinen als problematisch. Des weiteren sind die von der Leiterplatte wegführenden Kabel bei diesem Bildaufnehmermodul auf einer Außenseite der Leiterplatten mit dieser kontaktiert. Aufgrund des Durchmessers der einzelnen Kabel, die zum Teil auch Koaxialleitungen sein können und dann eine entsprechende Dicke besitzen, führt die Bauweise dieses bekannten Bildaufnehmermoduls zu einem Querschnittsdurchmesser, der größer ist als der durch den Bildsensor vorgegebene Querschnittsdurchmesser.

Ein bei der Verwendung derartiger Bildaufnehmermodule in Videoendoskopen ebenfalls auftretendes Problem besteht darin, daß das oder die von der Leiterplatte wegführenden Kabel einer Zugbeanspruchung ausgesetzt sein können, insbesondere bei flexiblen Videoendoskopen oder wenn, wie in der US 5,166,787 beschrieben ist, ein Ablenkmechanismus für das Bildaufnehmermodul zum Bewegen desselben vorgesehen ist, oder auch beim Einbau des Moduls in den Endoskopschaft. Auch hier erweist sich das aus der US 5,754,313 bekannte Bildaufnehmermodul als nachteilig, da hier die Zugentlastung entweder umfänglich um den Kontaktierungsbereich der Kabel an den beiden Leiterplatten vorgesehen werden müßte, wodurch sich der Querschnittsdurchmesser dieses bekannten Bildaufnehmermoduls nachteiligerweise weiter erhöhen würde, oder die Zugentlastung müßte proximalseitig der Leiterplatten erfolgen, was zu einer zusätzlichen, aber unerwünschten Verlängerung des Bildaufnehmermoduls führen würde, was speziell bei flexiblen Endoskopen, bei denen die steifen distalen Schnabelteile möglichst kurz sein sollen, problematisch ist.

Das aus der US 5,220,198 bekannte Bildaufnehmermodul weist eine einstückige Leiterplatte auf, die drei Abschnitte aufweist, die dort zusammen die Form eines U bilden, worauf die vorliegende Erfindung jedoch nicht beschränkt ist. Die zwei Abschnitte, die mit dem Bildsensor kontaktiert sind, verlaufen parallel zueinander, wobei der Bildsensor auf dem dritten Abschnitt, der quer zu den beiden anderen Abschnitten verläuft, befestigt ist. Die Leiterplatte ist demnach auf der dem Bildsensor abgewandten Seite offen. Auch bei diesem Bildaufnehmermodul besteht der Nachteil darin, daß für die von der Leiterplatte wegführenden Kabel keine Zugentlastungsmaßnahme vorgesehen ist. Bei Zugbeanspruchung der von der Leiterplatte wegführenden Kabel besteht somit die Gefahr, daß das oder die Kabel bei Zugbelastung von der Leiterplatte abreißen.

Bei dem aus der eingangs genannten JP 05 115463 A offenbarten Bildaufnehmermodul ist die einstückige Leiterplatte, die mit dem Bildsensor elektrisch kontaktiert ist, aus drei Abschnitten aufgebaut, von denen ein erster Abschnitt und ein zweiter Abschnitt voneinander beabstandet quer zu dem Bildsensor verlaufen und ein dritter Abschnitt zwischen dem ersten und dem zweiten Abschnitt angeordnet ist, wobei der Bildsensor an dem dem dritten Abschnitt gegenüberliegenden Ende der Leiterplatte angeordnet ist. Ein weiteres vergleichbares Bildaufnehmermodul ist aus der JP 06 178757 A bekannt.

Der Erfindung liegt daher die Aufgabe zugrunde, ein Bildaufnehmermodul der eingangs genannten Art dahingehend weiterzubilden, daß mit geringem konstruktivem Aufwand eine Zugentlastung für das zumindest eine von der Leiterplatte wegführende Kabel geschaffen wird, wobei die Zugentlastung die baulichen Abmessungen des Bildaufnehmermoduls nicht vergrößern soll.

Erfindungsgemäß wird diese Aufgabe hinsichtlich des eingangs genannten Bildaufnehmermoduls dadurch gelöst, daß der dritte Abschnitt der Zugentlastung des zumindest einen Kabels dient.

Anstatt nun wie bei dem aus der US 5,220,198 bekannten Bildaufnehmermodul die Leiterplatte mit dem Bildsensor derart zu kontaktieren, daß der Bildsensor auf dem dritten Abschnitt der Leiterplatte zu liegen kommt und die Leiterplatte auf der dem Bildsensor gegenüberliegenden Seite somit offen ist, ist erfindungsgemäß die Leiterplatte mit dem Bildsensor so kontaktiert, daß der Bildsensor an dem Ende der Leiterplatte, das dem dritten Abschnitt gegenüberliegt, angeordnet ist, so daß der dritte Abschnitt einen dem Bildsensor gegenüberliegenden Boden bildet. Der dritte Abschnitt wird vorteilhaft für eine Zugentlastung des zumindest einen von der Leiterplatte wegführenden Kabels genutzt. Der weitere Vorteil der erfindungsgemäßen Bauart des Bildaufnehmermoduls besteht darin, daß Zugentlastungsmaßnahmen an dem dritten Abschnitt ohne Vergrößerung der umfänglichen Abmessungen des Bildaufnehmermoduls über den Umfang des Bildsensors hinaus vorgesehen werden können, da der dritte Abschnitt zwischen dem ersten und zweiten Abschnitt angeordnet ist und diesen somit nicht überragt. Darüber hinaus kann der dritte Abschnitt vorteilhaft die Funktion der Kontaktierung zwischen dem ersten und zweiten Abschnitt erfüllen.

Der Erfindung liegt im Hinblick auf das eingangs genannte Verfahren ferner die Aufgabe zugrunde, das eingangs genannte Verfahren dahingehend zu verbessern, daß die Konfektionierung der Signalelektronik auf der Leiterplatte und auch das Kontaktieren mit dem zumindest einen Kabel erleichtert wird und auf einfache Weise eine Zugentlastung vorgesehen werden kann.

Erfindungsgemäß wird diese Aufgabe hinsichtlich des eingangs genannten Verfahrens dadurch gelöst, daß zunächst die Leiterplatte als planarer Plattenrohling vorliegt, der zumindest drei Abschnitte aufweist, die entlang flexibler Verbindungsabschnitte faltbar sind, daß das zumindest eine Kabel mit dem Plattenrohling kontaktiert wird, daß anschließend der Plattenrohling derart gefaltet wird, daß ein dritter Abschnitt zwischen einem ersten Abschnitt und einem zweiten Abschnitt angeordnet ist, und daß anschließend der Bildsensor an einem dem dritten Abschnitt gegenüberliegenden Ende der Leiterplatte mit dieser kontaktiert wird, wobei der dritte Abschnitt zur Zugentlastung des zumindest einen Kabels verwendet wird.

Erfindungsgemäß wird demnach nach dem Aufbringen der Signalelektronik auf dem Plattenrohling zunächst das zumindest eine Kabel mit der als planarer Plattenrohling vorliegenden Leiterplatte kontaktiert, was besonders einfach ist, da die einzelnen Kontakte auf der flach ausgebreiteten Leiterplatte leicht zugänglich sind.

Wenn das zumindest eine oder die mehreren Kabel mit dem planaren Plattenrohling kontaktiert sind, wird der Plattenrohling entlang der flexiblen Verbindungsabschnitte gefaltet, so daß der erste, zweite und dritte Abschnitt der zumindest drei Abschnitte die gewünschte Form bilden, wonach dann die gefaltete Struktur mit dem Bildsensor kontaktiert wird. Das erfindungsgemäße Verfahren ist daher insbesondere für die Herstellung miniaturisierter Bildaufnehmermodule geeignet.

In einer bevorzugten Ausgestaltung verlaufen der erste und der zweite Abschnitt etwa parallel zueinander und verläuft der dritte Abschnitt schräg oder quer zu dem ersten und zweiten Abschnitt.

Bei dieser Ausgestaltung der Leiterplatte weist diese etwa die Form eines U auf. Da in diesem Fall der dritte Abschnitt etwa quer zu dem von der Leiterplatte wegführenden zumindest einen Kabel verläuft, hat diese Ausgestaltung der Leiterplatte den Vorteil, daß der dritte Abschnitt auf das zumindest eine Kabel wirkende Zugkräfte im Sinne einer Zugentlastung wirksam aufnehmen kann.

Alternativ dazu ist es jedoch ebenso bevorzugt, wenn der dritte Abschnitt etwa V-förmig ausgebildet ist.

Hierbei ist von Vorteil, daß das zumindest eine von der Leiterplatte wegführende Kabel insbesondere auf der Außenseite des V-förmigen dritten Abschnitts kontaktiert werden kann, wodurch das Kontaktieren des Kabels an der Leiterplatte besonders einfach ist. Auch können ohne Umfangsvergrößerung des Bildaufnehmermoduls bei dieser Ausgestaltung Zugentlastungsmaßnahmen am dritten Abschnitt der Leiterplatte außenseitig vorgesehen werden, ohne den Außenumfang des Bildaufnehmermoduls über den Außenumfang des Bildsensors hinaus zu vergrößern. Ein weiterer besonderer Vorteil, der sich hieraus ergibt, ist die erreichbare besonders kurze Baulänge des gesamten Bildaufnehmermoduls.

Dabei können zwei Schenkel des V-förmigen dritten Abschnitts in geradliniger Verlängerung des ersten bzw. zweiten Abschnitts verlaufen.

Mit anderen Worten weist bei dieser Ausgestaltung die Leiterplatte insgesamt die Form eines V auf, ohne daß ein Übergang zwischen dem ersten Abschnitt und dem dritten Abschnitt bzw. dem zweiten Abschnitt und dem dritten Abschnitt erkennbar ist.

Alternativ dazu ist es jedoch auch bevorzugt, wenn der erste und der zweite Abschnitt etwa parallel zueinander verlaufen, so daß in diesem Fall der V-förmige dritte Abschnitt, genauer gesagt dessen zwei zusammenlaufende Schenkel, einen winkligen Übergang zum ersten bzw. zweiten Abschnitt bilden.

In einer weiteren bevorzugten Ausgestaltung ist es ebenso bevorzugt, wenn der dritte Abschnitt nach außen oder nach innen gewölbt ist. Auch dies kann im Sinne einer einfachen Herstellung und konstruktiv einfachen Ausgestaltung der Leiterplatte vorteilhaft sein.

In einer weiteren bevorzugten Ausgestaltung ist das zumindest eine Kabel auf einer innenliegenden Seite der Leiterplatte kontaktiert.

Hierbei ist von Vorteil, daß das zumindest eine Kabel im Innenbereich der gefalteten Leiterplatte angeordnet ist, so daß das Kabel, insbesondere bei Verwendung eines Koaxialkabels, oder bei einer Mehrzahl von Kabeln diese nicht zu einer Erhöhung der Außenabmessung des Bildaufnehmermoduls führen. Bei dem zuvor genannten erfindungsgemäßen Verfahren wird entsprechend nach dem Kontaktieren des zumindest einen Kabels der Plattenrohling so gefaltet, daß das Kabel im Innenbereich der gefalteten Leiterplatte zu liegen kommt.

Alternativ hierzu ist es insbesondere in der Ausgestaltung des dritten Abschnittes in Form eines V vorteilhaft, wenn das zumindest eine Kabel auf einer außenliegenden Seite der Leiterplatte, insbesondere auf der Außenseite des V-förmigen dritten Abschnitts, kontaktiert ist, wie bereits oben im Zusammenhang mit der Ausgestaltung des dritten Abschnitts in Form eines V beschrieben wurde.

In einer weiteren bevorzugten Ausgestaltung des Bildaufnehmermoduls weist der dritte Abschnitt zumindest einen Durchgang für das zumindest eine von der Leiterplatte wegführende Kabel auf.

Der Durchgang im dritten Abschnitt der Leiterplatte, der den Boden des Bildaufnehmermoduls bildet, hat insbesondere in der zuvor genannten Ausgestaltung den Vorteil, daß das innenseitig mit der Leiterplatte kontaktierte Kabel an dem dritten Abschnitt vorbei geführt ist, ohne daß das Kabel seitlich über die Außenabmessung der Leiterplatte übersteht und somit nicht zu einer Erhöhung der Außenabmessung des Bildaufnehmermoduls führt. Der zumindest eine Durchgang kann außerdem vorteilhaft als Zugentlastungsmaßnahme dienen, beispielsweise indem das Kabel oder die mehreren Kabel in dem zumindest einen Durchgang an dem dritten Abschnitt durch entsprechende Maßnahmen, beispielsweise Klemmen, lagefixiert werden. Der Durchgang kann als Durchgang für das gesamte Kabelsystem dienen, es können auch mehrere Durchgänge für einzelne der Kabel oder einzelne Kabelbündel vorgesehen sein.

Bei dem erfindungsgemäßen Verfahren wird der zumindest eine Durchgang bevorzugt vor dem Kontaktieren des zumindest einen Kabels ausgespart.

Dabei ist es weiterhin bevorzugt, wenn der zumindest eine Durchgang als randseitige Aussparung im dritten Abschnitt ausgebildet ist.

Hierbei ist von Vorteil, daß das zumindest eine Kabel zum Kontaktieren mit der Leiterplatte der Länge nach ausgestreckt auf der Leiterplatte positioniert werden kann und sich der dritte Abschnitt beim Falten dann mühelos an dem Kabel vorbei in seine quer zu dem wegführenden Kabel verlaufende Endposition falten läßt, ohne daß das Kabel ein Hindernis beim Falten darstellt.

Es ist jedoch auch bevorzugt, wenn der zumindest eine Durchgang als etwa mittige Öffnung im dritten Abschnitt ausgebildet ist.

Bei dieser Ausgestaltung muß zwar vor dem Kontaktieren des zumindest einen Kabels dieses durch den planar ausgebreiteten Plattenrohling hindurchgefädelt werden, jedoch hat diese Maßnahme den Vorteil, daß das zumindest eine wegführende Kabel oder das Kabelbündel mittig von der Leiterplatte weggeführt werden kann.

In einer weiteren bevorzugten Ausgestaltung des Bildaufnehmermoduls ist der durch den ersten, zweiten und dritten Abschnitt aufgespannte Innenbereich der Leiterplatte mit einer elektrisch nichtleitenden Füllmasse ausgefüllt.

Diese Maßnahme stellt eine besonders vorteilhafte Art einer Zugentlastung des zumindest einen wegführenden Kabels dar, da die Füllmasse eine innige Verbindung zwischen den Abschnitten der Leiterplatte und dem zumindest einen wegführenden Kabel herstellt, die auch besonderes hohen Zugkräften sicher standhält. Ein weiterer Vorteil besteht darin, daß die Zugentlastung in diesem Fall vollständig im Innenbereich der Leiterplatte erfolgt, so daß keine Abmessungsvergrößerung des Bildaufnehmermoduls auftritt. Außerdem verfestigt die Füllmasse den Gesamtaufbau in vorteilhafter Weise.

Bei dem erfindungsgemäßen Verfahren wird demnach der durch den ersten, zweiten und dritten Abschnitt aufgespannte Innenbereich der gefalteten Leiterplatte mit einer aushärtenden elektrisch nichtleitenden Füllmasse ausgefüllt und anschließend der Bildsensor mit der Leiterplatte kontaktiert.

Auf diese Weise läßt sich die Zugentlastung verfahrensmäßig besonders einfach implementieren, da eine aushärtende Füllmasse im weichen oder gar fließfähigen Zustand mühelos in den Innenbereich der Leiterplatte eingebracht werden kann.

Als Füllmasse kann ein aushärtendes Harz, beispielsweise ein Zweikomponentenklebstoff verwendet werden. Dazu kann die gefaltete Leiterplatte in eine Form oder Verschalung eingesetzt werden, um ein Entweichen der noch nicht ausgehärteten Füllmasse zu verhindern.

In einer weiteren bevorzugten Ausgestaltung des Bildaufnehmermoduls weist die Leiterplatte einen vierten Abschnitt auf, der dem dritten Abschnitt gegenüberliegend angeordnet ist, und auf dessen Außenseite der Bildsensor zu liegen kommt.

Der vierte Abschnitt dient demnach vorteilhaft als flächige Auflage bzw. Trägerplatte für den Bildsensor, so daß dieser außer über die Kontaktierung an der Außenseite des ersten und zweiten Abschnitts zusätzlich mechanisch sicher mit der Leiterplatte verbunden werden kann. Der vierte Abschnitt kann außerdem vorteilhaft zusätzliche Bauelemente aufnehmen, steht jedoch gegebenenfalls nur mechanisch und normalerweise nicht elektrisch mit dem Bildsensor in Verbindung.

Im Falle des erfindungsgemäßen Verfahrens, nach dem die Leiterplatte aus einem planaren Plattenrohling gefaltet wird, kann der Plattenrohling auch den vierten Abschnitt aufweisen, der mit einem oder mehreren der bereits genannten drei Abschnitten einstückig flexibel verbunden ist, so daß der Aufwand beim Zusammenbauen dadurch nicht erhöht ist.

Dabei ist es außerdem bevorzugt, wenn der vierte Abschnitt zumindest ein elektrisches Bauteil und/oder zumindest eine elektrische Leiterbahn aufweist.

Hierbei ist von Vorteil, daß auch der vierte Abschnitt eine Möglichkeit zum Aufnehmen von Teilen der Signalelektronik bietet, so daß für die Schaltungen und elektronischen Bauteile insgesamt mehr Platz zur Verfügung steht.

In einer weiteren bevorzugten Ausgestaltung weist die Leiterplatte zusätzlich zu dem ersten Abschnitt und dem zweiten Abschnitt einen weiteren Abschnitt oder zwei weitere Abschnitte auf, der oder die an einer Längsseite der Leiterplatte angeordnet ist/sind.

Diese Maßnahme hat den Vorteil, daß die gesamte Signalelektronik der Leiterplatte gehäuseartig allseitig eingekapselt und damit gegen Beschädigung geschützt werden kann. Ein weiterer Vorteil dieser Maßnahme besteht im Zusammenhang mit dem Ausfüllen des Innenbereichs der Leiterplatte mit einem aushärtenden Füllstoff darin, daß durch die allseitige Einfassung des Innenbereichs der Leiterplatte auch ohne Verschalungsmaßnahmen ein Wegfließen der Füllmasse vor ihrem Aushärten weitestgehend vermieden werden kann.

In einer weiteren bevorzugten Ausgestaltung weist die Leiterplatte auf ihrer außenliegenden Seite Vertiefungen zur Kontaktierung des Bildsensors auf.

Da üblicherweise die Kontaktfinger des Bildsensors auf den Außenseiten der Leiterplatte mit dieser kontaktiert werden, hat diese Maßnahme den Vorteil, daß die Kontaktfinger in die Vertiefungen eingelegt werden können, wodurch sich die Außenabmessungen durch die Kontaktierung des Bildsensors in der Leiterplatte nicht erhöhen.

In einer weiteren bevorzugten Ausgestaltung weist der dritte Abschnitt der Leiterplatte zumindest einen Kontakt zum Kontaktieren des von der Leiterplatte wegführenden Kabels auf.

Im Zusammenhang mit der erfindungsgemäßen Ausgestaltung, wonach die Leiterplatte einen Boden in Form des dritten Abschnitts aufweist, kann dieser vorteilhaft zum Kontaktieren weiterer Kabel oder einer Schirmleitung für das gesamte gegebenenfalls mehradrige Kabel dienen, wobei diese Kabel an dem dritten Abschnitt ebenfalls durch entsprechende Maßnahmen zugentlastet kontaktiert werden können.

In einer weiteren bevorzugten Ausgestaltung weist der dritte Abschnitt der Leiterplatte zumindest eine elektrische Leiterbahn zum elektrischen Verbinden des ersten Abschnitts und des zweiten Abschnitts auf.

Hierbei ist von Vorteil, daß der dritte Abschnitt außer der Funktion einer Zugentiastung zusätzlich noch die Funktion einer elektrischen Verbindung zwischen dem ersten und dem zweiten Abschnitt haben kann, ohne daß für diese elektrische Verbindung zusätzliche Leitungen oder Verbindungsleiterplatten erforderlich sind.

Ein besonders bevorzugter Plattenrohling, aus dem die Leiterplatte gefaltet wird, ist so zugeschnitten, daß der erste Abschnitt und der zweite Abschnitt über einen weiteren Abschnitt flexibel verbunden, jedoch beabstandet auf gleicher Höhe angeordnet sind, daß der erste Abschnitt und der zweite Abschnitt zum Kontaktieren jeweils zumindest eines Kabels dienen, und daß der dritte Abschnitt an einer Stirnseite des weiteren Abschnitts flexibel mit diesem verbunden ist.

Mit einem derartig zugeschnittenen Plattenrohling gestaltet sich das Konfektionieren der Signalelektronik und vor allem das Kontaktieren von mehreren Kabeln besonders einfach, da auf dem ersten und dem zweiten Abschnitt jeweils ein Kabelbündel beabstandet voneinander und sich damit nicht gegenseitig behindernd kontaktiert werden kann, und auch der anschließende Faltvorgang zum Fertigstellen der Leiterplatte in die zu bildende Struktur gestaltet sich besonders einfach, insbesondere wenn in dem dritten Abschnitt die Durchgänge als randseitige Aussparungen vorgesehen sind.

Anstatt der bereits erwähnten Maßnahme, den durch den ersten, zweiten und dritten Abschnitt aufgespannten Innenbereich der Leiterplatte mit einem Füllstoff auszufüllen, kann auch vorgesehen sein, diesen Innenbereich durch einen Kontaktkörper auszufüllen, der zum einen den Kontakt zu dem ersten Abschnitt und dem zweiten Abschnitt herstellt und zum anderen ein Steckersystem für das zumindest eine wegführende Kabel beinhaltet. Ein solcher Stecker innerhalb des Kontaktkörpers ist bevorzugt ein Mikrostecker-System. Derartige Mikrostecker können zwischenzeitlich durch die Fortschritte in der Mikrotechnologie hergestellt werden.

Weitere Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf diese näher beschrieben. Es zeigen:
- Fig. 1: ein Bildaufnehmermodul in einer perspektivischen Darstellung gemäß einem ersten Ausführungsbeispiel;
- Fig. 2: einen Schnitt entlang einer Linie II-II in Fig. 1;
- Fig. 3: einen Plattenrohling, aus dem die Leiterplatte des Bildaufnehmermoduls in Fig. 1 und 2 gefaltet wird, in Draufsicht;
- Fig. 4: ein Bildaufnehmermodul in einer perspektivischen Darstellung gemäß einem zweiten Ausführungsbeispiel;
- Fig. 5: einen Schnitt entlang einer Linie V-V in Fig. 4;
- Fig. 6: einen Plattenrohling, aus dem die Leiterplatte des Bildaufnehmermoduls in Fig. 4 und 5 gefaltet wurde, in Draufsicht;
- Fig. 7: ein Bildaufnehmermodul in einer perspektivischen Darstellung gemäß einem dritten Ausführungsbeispiel;
- Fig. 8: eine Seitenansicht des Bildaufnehmermoduls in Fig. 7;
- Fig. 9: einen Schnitt entlang einer Linie IX-IX in Fig. 8;
- Fig. 10: einen Plattenrohling, aus dem die Leiterplatte des Bildaufnehmermoduls in Fig. 7 bis 9 gefaltet wurde, in Draufsicht;
- Fig. 11: ein Bildaufnehmermodul in einer perspektivischen Darstellung gemäß einem vierten Ausführungsbeispiel;
- Fig. 12: einen Plattenrohling, aus dem die Leiterplatte des Bildaufnehmermoduls in Fig. 11 gefaltet wurde, in Draufsicht; und
- Fig. 13: ein Bildaufnehmermodul im Längsschnitt gemäß einem fünften Ausführungsbeispiel.

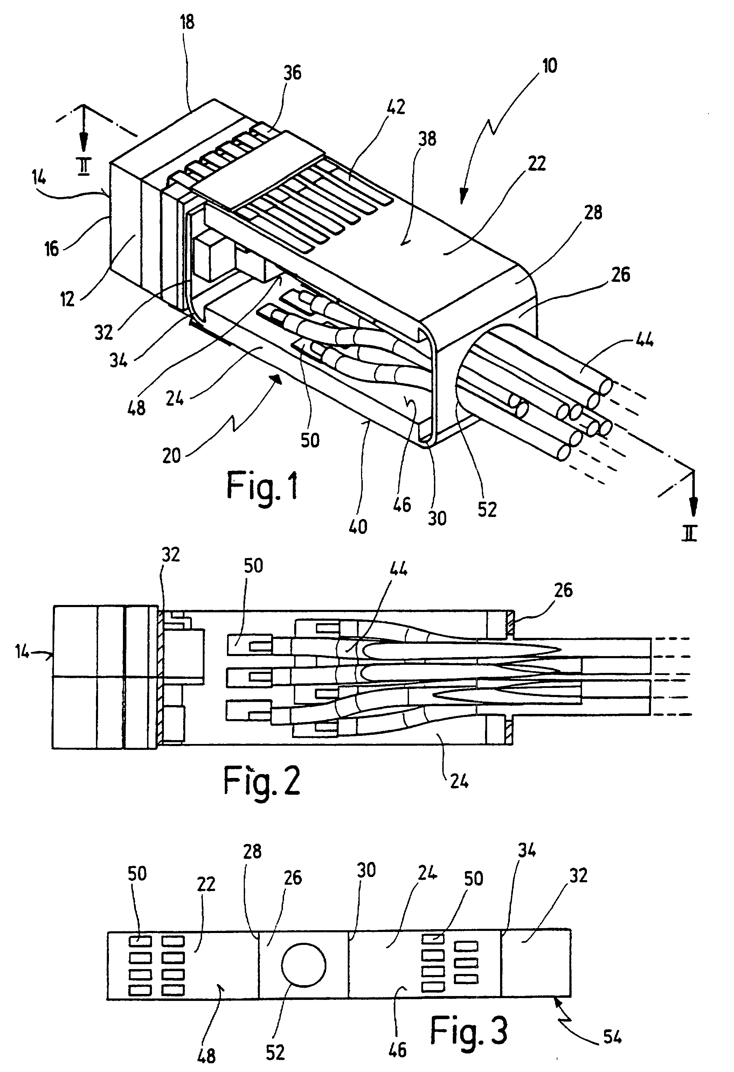

In Fig. 1 ist ein mit dem allgemeinen Bezugszeichen 10 versehenes elektronisches Bildaufnehmermodul dargestellt. Das Bildaufnehmermodul 10 wird beispielsweise in die distale Spitze eines nicht dargestellten Endoskopschafts eingebaut. Das in Fig. 1 dargestellte Bildaufnehmermodul 10 ist, so wie es dargestellt ist, einbaufertig, d.h. das Bildaufnehmermodul 10 wird nicht noch in ein weiteres Gehäuse eingekapselt, was bei der Ausgestaltung des erfindungsgemäßen Bildaufnehmermoduls 10 auch nicht erforderlich ist, wie aus der nachfolgenden Beschreibung hervorgeht, da alle empfindlichen und möglicherweise zugbelasteten Teile sicher geschützt sind.

Das Bildaufnehmermodul 10 weist einen Bildsensor 12 auf, der eine Bildaufnahmefläche 14 aufweist.

Der Bildsensor 12 ist ein elektronischer Bildsensor in CCDoder CMOS-Ausführung. Ein derartiger Bildsensor ist ein handelsüblicher Bildsensor. Ein solcher Bildsensor stellt einen optoelektronischen Wandler dar, d.h. über die Bildaufnahmefläche 14 pixelweise empfangenes Licht wird von dem Bildsensor 12 in elektrische Signale umgewandelt.

Der Bildsensor 12 ist insbesondere ein miniaturisierter Bildsensor, dessen äußere Kanten 16 und 18 eine Länge von jeweils etwa 4 mm aufweisen, um ein Beispiel zu nennen. Der Bildsensor 12 ist weiterhin gehäuselos in sogenannter TAB-Ausführung ausgestaltet, dessen Kontakte seitlich aus dem Sensor herausstehen. Die gehäuselose Ausgestaltung des Bildsensors 12 trägt weiter zu einer platzsparenden Bauweise des Bildaufnehmermoduls bei.

Das Bildaufnehmermodul 10 weist weiterhin eine mit dem allgemeinen Bezugszeichen 20 versehene Leiterplatte auf. Die Leiterplatte 20 ist insgesamt einstückig ausgebildet.

Die Leiterplatte 20 weist einen ersten Abschnitt 22 und einen zweiten Abschnitt 24 auf. Der erste Abschnitt 22 und der zweite Abschnitt 24 verlaufen beabstandet voneinander etwa parallel zueinander und etwa senkrecht zum Bildsensor 12 bzw. zur Bildaufnahmefläche 14.

Die Leiterplatte 20 weist weiterhin einen dritten Abschnitt 26 auf, der quer zu dem ersten Abschnitt 22 und quer zu dem zweiten Abschnitt 24 und etwa parallel zum Bildsensor 12 bzw. zur Bildaufnahmefläche 14 verläuft. Der dritte Abschnitt 26 verbindet den ersten Abschnitt 22 und den zweiten Abschnitt 20 in diesem Fall einstückig miteinander.

Dabei ist der dritte Abschnitt 26 mit dem ersten Abschnitt 22 über einen ersten flexiblen Verbindungsabschnitt 28 und mit dem zweiten Abschnitt 24 durch einen zweiten flexiblen Verbindungsabschnitt 30 einstückig verbunden. Wie aus Fig. 1 hervorgeht, weisen der erste Abschnitt 22 und der zweite Abschnitt 24 eine größere Dicke auf als der dritte Abschnitt 26 und als die Verbindungsabschnitte 28 und 30.

Die Leiterplatte 20 weist weiterhin einen vierten Abschnitt 32 auf, der über einen dritten flexiblen Verbindungsabschnitt 34 mit dem zweiten Abschnitt 24 einstückig flexibel verbunden ist. Der vierte Abschnitt 32 verläuft etwa parallel zu dem dritten Abschnitt 26 und ist diesem gegenüberliegend am anderen Ende der Leiterplatte 20 angeordnet.

Der erste Abschnitt 22, der zweite Abschnitt 24 und der dritte Abschnitt 26 bilden zusammen die Form eines U.

Die Leiterplatte 20, hier genauer der erste Abschnitt 22 und der zweite Abschnitt 24 der Leiterplatte 20, nimmt mikroelektronische Bauelemente, elektrische Leiterbahnen, aufgedruckte elektrische Schaltungen und dergleichen auf, die die Signalelektronik des Bildsensors 12 bilden.

Der Bildsensor 12 ist mit der Leiterplatte 20, genauer gesagt mit dem ersten Abschnitt 22 und dem zweiten Abschnitt 24, elektrisch kontaktiert. Dazu weist der Bildsensor 12 eine Mehrzahl von Kontaktfingern 36 auf, wobei in dem gezeigten Ausführungsbeispiel der Bildsensor 12 fünf Kontaktfinger 36 und diesen gegenüberliegend weitere fünf Kontaktfinger aufweist, die auf gegenüberliegenden Seiten aus dem Bildsensor 12 nach außen treten. Die Kontaktierung des Bildsensors 12 mit der Leiterplatte 20 erfolgt dabei auf einer Außenseite 38 des ersten Abschnitts 22 bzw. auf einer dieser gegenüberliegenden Außenseite 40 des zweiten Abschnitts 24 der Leiterplatte 20, wobei auf den Außenseiten 38 und 40 jeweils entsprechende Kontaktflächen 42 vorhanden sind. Die Kontaktflächen 42 sind dabei in Vertiefungen in den Außenseiten 38 und 40 angeordnet, so daß die Kontaktfinger 36 des Bildsensors 12 in diesen Vertiefungen liegen und somit nicht seitlich nach außen überstehen.

Mit der Leiterplatte 20 ist weiterhin zumindest ein von der Leiterplatte 20 wegführendes elektrisches Kabel 44 kontaktiert, wobei im gezeigten Ausführungsbeispiel ein Kabelsystem aus einer Vielzahl von Kabeln 44 von der Leiterplatte 20 wegführt. Die Kabel 44 führen zu einer fern liegenden nicht dargestellten Signalverarbeitungs- bzw. Ansteuerungselektronik für das Bildaufnehmermodul 10 und können von einem Mantel, beispielsweise einem Kunststoffmantel, zusammengefaßt werden.

Die Kontaktierung der Kabel 44 erfolgt dabei auf Innenseiten 46 und 48 des zweiten Abschnitts 24 bzw. des ersten Abschnitts 22 der Leiterplatte 20, wozu die Innenseiten 46 und 48 entsprechende Kontaktflächen 50 aufweisen, die ebenfalls wie die Kontaktflächen 42 auf den Außenseiten 38 und 40 in entsprechenden Vertiefungen angeordnet sind.

Da, wie bereits erwähnt, der dritte Abschnitt 26 dem Bildsensor 12 gegenüberliegend angeordnet ist, bildet der dritte Abschnitt 26 einen Boden, der eine Zugentlastung ohne zusätzliche raumgreifende, die Außenabmessung des Bildaufnehmermoduls 10 vergrößernde Maßnahmen ermöglicht.

In dem dritten Abschnitt 26 ist zunächst ein Durchgang 52 in Form einer etwa mittigen Öffnung ausgespart, durch die die Kabel 44 durchgeführt sind. Eine erste Zugentlastung wird durch das Durchführen der Kabel 44 durch den Durchgang 52 erreicht, da die Kabel 44 in dem Durchgang 52 aufgrund der Haftreibung der äußeren Kabel 44 am Rand des Durchgangs 52 und der Haftreibung der einzelnen Kabel 44 untereinander in ihrer Längsrichtung an einer Verschiebung gehemmt sind.

Eine gegen besonders hohe Zugbeanspruchungen wirksame Zugentlastung wird bei dem Bildaufnehmermodul 10 dadurch erreicht, daß der durch den ersten Abschnitt 22, den zweiten Abschnitt 24 und den dritten Abschnitt 26 aufgespannte Innenbereich der Leiterplatte 20, in dem die Enden der Kabel 44 mit der Leiterplatte 20 kontaktiert sind, mit einem nichtleitenden Füllmaterial ausgefüllt ist, das eine besonders innige Verbindung der Kabel 44 mit der Leiterplatte 22 bewerkstelligt, indem der Füllstoff alle oder nahezu alle verbleibenden Hohlräume innerhalb der Leiterplatte ausfüllt. Die Füllmasse ist beispielsweise ein ausgehärteter Harz, beispielsweise ein Zweikomponentenkleber, der elektrisch nicht leitend ist.

Wie aus Fig. 1 weiter hervorgeht, ist der Bildsensor 12 auf dem vierten Abschnitt 32 der Leiterplatte 20 angeordnet, so daß der vierte Abschnitt 32 als zusätzliche Halterung des Bildsensors 12 an der Leiterplatte 22 dient.

In Fig. 3 ist ein Plattenrohling 54 dargestellt, aus dem die Leiterplatte 20 des Bildaufnehmermoduls 10 in Fig. 1 und 2 gefaltet wurde.

Bei einem Verfahren zum Zusammenbauen der Bildaufnehmereinheit 10 wird dabei wie folgt vorgegangen. Ausgehend von dem planaren Plattenrohling 54 werden zunächst auf den ersten Abschnitt 22 und den zweiten Abschnitt 24 auf deren späteren Innenseiten 48 und 46 (in Fig. 3 die Seite der Draufsicht) die mikroelektronischen Bauteile, Leiterbahnen und dergleichen aufgebracht. Weiterhin im entfalteten Zustand, d.h. im planaren Zustand des Plattenrohlings 54, werden dann die Kabel 44 mit dem ersten Abschnitt 22 und dem zweiten Abschnitt 24 an den Kontaktflächen 50 kontaktiert. Zuvor wurden die entsprechenden Kabelenden der Kabel 44 durch den Durchgang 52 im dritten Abschnitt 26 der Leiterplatte 20 in Form der etwa mittigen Öffnung durchgeführt, die vor dem Kontaktieren in den dritten Abschnitt 26 des Plattenrohlings 54 ausgespart wurde.

Nach dem Kontaktieren aller Kabel 44 mit dem Plattenrohling 54 werden dann die Abschnitte 22, 24, 26 und 32 zu der in Fig. 1 dargestellten Form der Leiterplatte 20 gefaltet. Anschließend wird dann der Innenbereich der Leiterplatte 20 mit einer aushärtenden Füllmasse ausgehärtet, die im ausgehärteten Zustand den gesamten Aufbau des Bildaufnehmermoduls 10 verfestigt und eine zusätzliche Zugentlastung für die Kabel 44 gewährleistet. Schließlich wird dann der Bildsensor 12 mit der Leiterplatte 20 kontaktiert.

Der dritte Abschnitt 26 kann ebenfalls noch elektrische Leiterbahnen aufweisen, die den ersten Abschnitt 22 und den zweiten Abschnitt 24 der Leiterplatte 20 elektrisch miteinander verbinden.

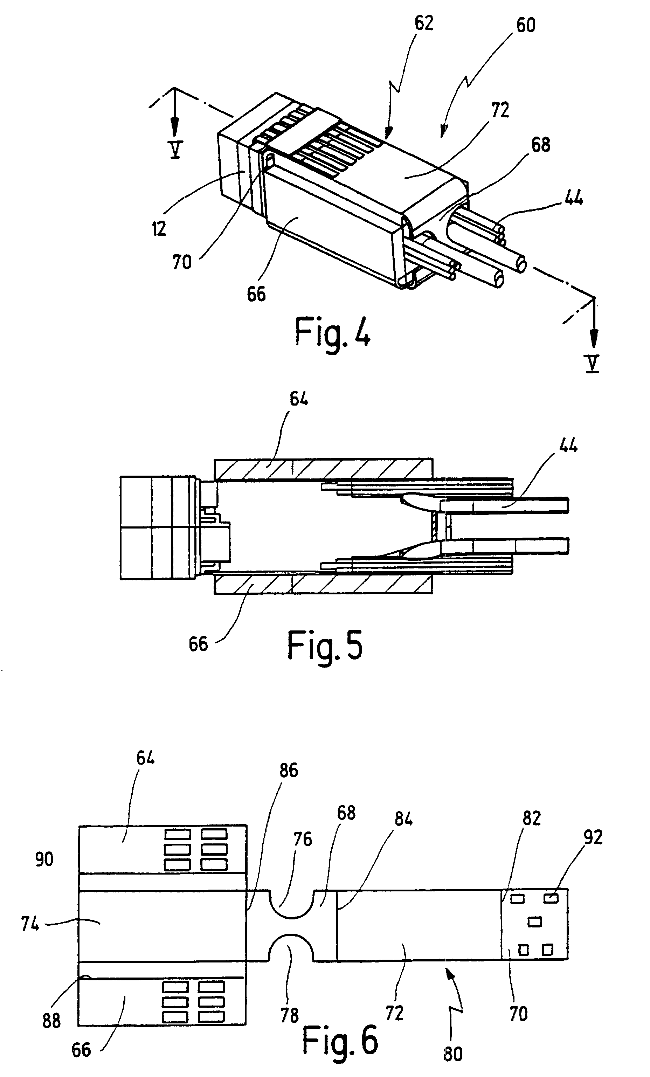

In Fig. 4 und 5 ist ein weiteres Ausführungsbeispiel eines Bildaufnehmermoduls 60 dargestellt, wobei nachfolgend hauptsächlich die Unterschiede zu dem vorherigen Ausführungsbeispiel beschrieben werden und nicht erwähnte Merkmale identisch oder gleichartig mit entsprechenden Merkmalen des Bildaufnehmermoduls 10 sind.

Hier ist der Bildsensor 12 mit einer Leiterplatte 62 kontaktiert, die einen ersten Abschnitt 64, einen zweiten Abschnitt 66 und einen dritten Abschnitt 68 aufweist, die zusammen die Form eines U bilden. Die Leiterplatte 62 ist ebenfalls insgesamt einstückig ausgebildet. Der dritte Abschnitt 68 ist dem Bildsensor 12 gegenüberliegend angeordnet. Ein vierter Abschnitt 70, der dem vierten Abschnitt 32 der Leiterplatte 20 in Fig. 1 bis 3 entspricht, dient wiederum als mechanische Halterung bzw. Unterstützung für den Bildsensor 12.

Die Leiterplatte 62 weist jedoch zusätzlich zu den vorgenannten Abschnitten noch einen fünften Abschnitt 72 und einen sechsten Abschnitt 74 auf, die den ersten Abschnitt 64 und den zweiten Abschnitt 66 als Seitenwände miteinander verbinden.

Der Bildsensor 12 ist dabei mit den Abschnitten 72 und 74 kontaktiert, während die Kabel 44 mit dem ersten Abschnitt 64 und dem zweiten Abschnitt 66 kontaktiert sind.

Die Leiterplatte 62 bildet somit ein allseitig bewandetes Gehäuse, in dem die Signalelektronik und die Kontaktierungen der Kabel 44 sicher geschützt sind. Auch bei diesem Ausführungsbeispiel kann der Innenbereich mit einer ausgehärteten Füllmasse ausgefüllt sein, um eine zusätzliche Zugentlastung für die Kabel 44 zu bilden.

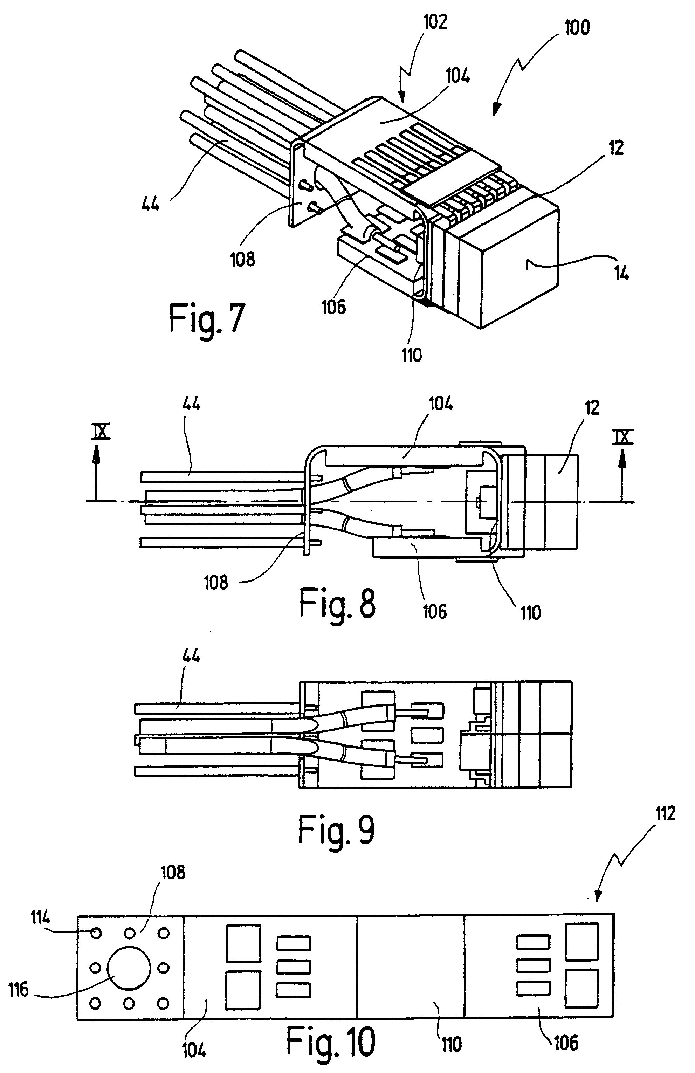

Der dritte Abschnitt 68 weist zumindest einen Durchgang, im gezeigten Ausführungsbeispiel zwei Durchgänge 76 und 78 für die Kabel 44 auf, die jedoch nicht als mittige Öffnung, sondern als randseitige Aussparungen ausgebildet sind, die, wie aus Fig. 6 hervorgeht, randseitig in dem dritten Abschnitt 68 ausgespart sind. Dies hat beim Zusammenbau des Bildaufnehmermoduls 60 den Vorteil, daß die Kabel 44 vor ihrer Kontaktierung mit dem ersten Abschnitt 64 und dem zweiten Abschnitt 66 nicht durch eine Öffnung hindurchgefädelt werden müssen.

Ein planarer Plattenrohling 80, aus dem die Leiterplatte 62 gefaltet ist, ist in Fig. 6 dargestellt.

Bei dem Plattenrohling 80 sind der erste Abschnitt 64 und der zweite Abschnitt 66 parallel zueinander auf gleicher Höhe angeordnet und durch den weiteren Abschnitt 74 beabstandet voneinander miteinander verbunden. Der dritte Abschnitt 68 ist an der Stirnseite des weiteren Abschnitts 74 mit diesem verbunden.

Bei diesem Plattenrohling 80 ist das Kontaktieren der Kabel 44 an dem ersten Abschnitt 64 und dem zweiten Abschnitt 66 besonders einfach, da die Kabel 44 in zwei parallelen Strängen mit ihrer Längsausrichtung in der Ebene des Plattenrohlings 80 gelegt werden können und nicht zuvor durch eine Öffnung hindurchgeführt werden müssen. Die Kabel 44 können also vollkommen ausgestreckt, d.h. ohne Biegung, auf die Arbeitsunterlage gelegt werden. Nach dem Kontaktieren können dann die einzelnen Abschnitte 64 bis 74 entlang flexibler Verbindungsabschnitte 82 bis 90 zu der in Fig. 4 gezeigten Struktur der Leiterplatte 62 gefaltet werden, wobei sich der dritte Abschnitt 68 dabei mühelos mit den Durchgängen 76 und 78 an den beiden Strängen der Kabel 44 vorbei falten läßt.

Bei diesem Ausführungsbeispiel ist weiterhin vorgesehen, daß der vierte Abschnitt 70 der Leiterplatte 62 ebenfalls Kontakte 92 aufweist.

In Fig. 7 bis 9 ist in einem weiteren Ausführungsbeispiel ein Bildaufnehmermodul 100 gemäß der vorliegenden Erfindung dargestellt, das sich bezüglich der vorherigen Ausführungsbeispiele durch einzelne Merkmale einer Leiterplatte 102 unterscheidet.

Die Leiterplatte 102 weist einen ersten Abschnitt 104, einen zweiten Abschnitt 106, einen dritten Abschnitt 108 und einen vierten Abschnitt 110 auf, die insgesamt einstückig miteinander verbunden sind, wobei der erste Abschnitt 104, der zweite Abschnitt 106 und der dritte Abschnitt 108 ein U bilden. Im Unterschied zu den beiden vorherigen Ausführungsbeispielen besteht jedoch zwischen dem dritten Abschnitt 108 und dem zweiten Abschnitt 106 keine direkte Verbindung. Ein zugehöriger planarer Plattenrohling 112, aus dem die Leiterplatte 102 entsprechend der in Fig. 7 und 8 dargestellten Struktur gefaltet ist, ist in Fig. 10 dargestellt.

Der dritte Abschnitt 108 der Leiterplatte 102 weist zusätzliche Kontaktierungsmöglichkeiten für einen Teil der Kabel 44 auf, wozu in dem dritten Abschnitt 108 zusätzliche Öffnungen 114 vorgesehen sind. Ein Durchgang 116 für die übrigen Kabel 44 ist ebenfalls wiederum in dem dritten Abschnitt 108 ausgespart, so daß dieser Teil der Kabel 44 mit den Abschnitten 104 und 106 der Leiterplatten 102 auf deren Innenseiten kontaktierbar ist.

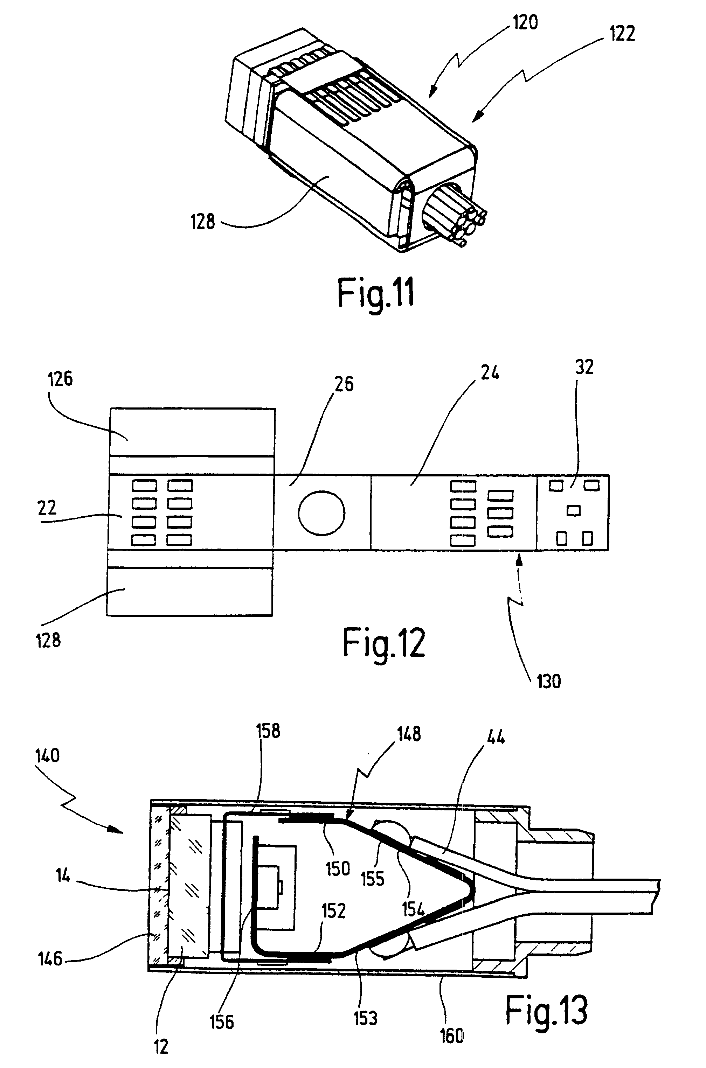

Ein viertes Ausführungsbeispiel ist schließlich in Fig. 11 und 12 dargestellt, das dem Ausführungsbeispiel in Fig. 1 bis 3 sehr ähnlich ist und eine Erweiterung dieses Ausführungsbeispiels darstellt. Hiernach weist ein Bildaufnehmermodul 120 eine einstückige Leiterplatte 122 auf, die zusätzlich zu den ersten vier Abschnitten 22, 24, 26, 32 in Fig. 1 noch einen fünften Abschnitt 124 und einen sechsten Abschnitt 128 aufweist, die mit dem ersten Abschnitt 22 gemäß Fig. 12 einstückig verbunden sind und einen seitlichen Abschluß der Leiterplatte 122 bilden. Ein entsprechender planarer Plattenrohling 130, aus dem die Leiterplatte 122 gefaltet ist, ist in Fig. 12 dargestellt.

In Fig. 13 ist ein noch weiteres Ausführungsbeispiel eines Bildaufnehmermoduls 140 dargestellt, das sich von den bisherigen Ausführungsbeispielen insbesondere durch folgende Merkmale unterscheidet:

Das Bildaufnehmermodul 140 weist eine Leiterplatte 148 auf, die, wie bisher, einen ersten Abschnitt 150 und einen zweiten Abschnitt 152 sowie einen dritten Abschnitt 154 aufweist.

Der dritte Abschnitt 154 ist im Unterschied zu den vorherigen dritten Abschnitten in Form eines V ausgebildet. Der dritte Abschnitt 154 weist einen ersten Schenkel 153 und einen zweiten Schenkel 155 auf, die zum dem Bildsensor 12 gegenüberliegenden Ende zusammenlaufen.

Der erste Schenkel 153 verläuft schräg zum zweiten Abschnitt 152 bzw. bildet mit diesem einen Winkel, während der zweite Schenkel 155 mit dem ersten Abschnitt 150 ebenfalls einen Winkel bildet. Alternativ dazu kann es jedoch auch vorgesehen sein, daß der erste Schenkel 153 mit dem zweiten Abschnitt 152 eine geradlinige Verlängerung bildet, d.h. daß in diesem Fall der zweite Abschnitt 152 ebenfalls schräg zum Bildsensor 12 verläuft, und ebenso kann der zweite Schenkel 155 des dritten Abschnitts 154 mit dem ersten Abschnitt 150 eine geradlinige Verlängerung bilden. In diesem Fall hätte dann die gesamte Leiterplatte 148 die Form eines V.

Wie aus Fig. 13 hervorgeht, ist das zumindest eine Kabel 44 an einer außenliegenden Seite der Leiterplatte 148 mit dieser kontaktiert, und zwar genauer auf einer außenliegenden Seite des V-förmigen dritten Abschnitts 154. Wie in Fig. 13 zu erkennen ist, stellt diese Kontaktierung des zumindest einen Kabels 44 auf der außenliegenden Seite des V-förmigen dritten Abschnitts 154 keine Umfangsvergrößerung des Bildaufnehmermoduls 140 über den Außenumfang des Bildsensors 12 dar. Es kann jedoch ebenso vorgesehen sein, das zumindest eine Kabel 44 auf einer innenliegenden Seite der Leiterplatte 148 mit dieser zu kontaktieren, wie dies in den vorherigen Ausführungsbeispielen beschrieben wurde.

Zusätzlich können Zugentlastungsmaßnahmen (nicht dargestellt) im Bereich der Außenseite des dritten Abschnitts 154 der Leiterplatte 148 vorgesehen sein, beispielsweise Klammern oder dergleichen. Eine weitere Zugentlastungsmaßnahme kann darin bestehen, den Innenraum eines Gehäuses 160, in dem das Bildaufnehmermodul 140 angeordnet ist, mit einer aushärtenden Füllmasse auszugießen.

Die Leiterplatte 148 weist ebenfalls wiederum einen vierten Abschnitt 156 auf, der weitere elektrische oder elektronische Bauelemente trägt oder tragen kann. Kontaktfinger 158, über die der Bildsensor 12 mit der Leiterplatte 148 auf dessen Außenseite kontaktiert ist, sind ebenfalls in Fig. 13 dargestellt. Ein Deckglas 146 schließt das distale Ende des Gehäuses 160 vor dem Bildsensor 12 ab.

Die Baulänge des Bildaufnehmermoduls 140 ist gegenüber den vorherigen Ausführungsbeispielen kürzer. Die vorhergehenden Ausführungsbeispiele zeigen, daß im Rahmen der vorliegenden Erfindung eine große Anzahl von Abwandlungen möglich sind, die jedoch allesamt den Vorteil einer Zugentlastung der von der Leiterplatte wegführenden Kabel ohne eine Vergrößerung der Außenabmessungen des jeweiligen Bildaufnehmermoduls bieten, was durch den bodenseitigen dritten Abschnitt der Leiterplatte erreicht wird.

## Patentansprüche

1. Bildaufnehmermodul, insbesondere für ein Endoskop, mit einem elektronischen Bildsensor (12), mit einer einstückigen Leiterplatte (20; 62; 102, 122; 148), die mit dem Bildsensor (12) elektrisch kontaktiert ist, und mit der weiterhin zumindest ein von der Leiterplatte (20; 62; 102, 122; 148) wegführendes Kabel (44) kontaktiert ist, wobei die Leiterplatte (20; 62; 102, 122; 148) zumindest drei Abschnitte (22, 24, 26; 64, 66, 68; 104, 106, 108; 150, 152, 154) aufweist, von denen ein erster Abschnitt (22; 64; 104; 150) und ein zweiter Abschnitt (24; 66; 106; 152) voneinander beabstandet schräg oder quer zu dem Bildsensor (12) verlaufen und ein dritter Abschnitt (26; 68; 108; 154) zwischen dem ersten und zweiten Abschnitt (22, 24; 64, 66; 104, 106) angeordnet ist, wobei der Bildsensor (12) an einem dem dritten Abschnitt (26; 68; 108; 154) gegenüberliegenden Ende der Leiterplatte (20; 62; 102; 122; 148) angeordnet ist, **dadurch gekennzeichnet, daß** der dritte Abschnitt (26; 68; 108; 154) der Zugentlastung des zumindest einen Kabels (44) dient.

2. Bildaufnehmermodul nach Anspruch 1, **dadurch gekennzeichnet, daß** der erste und der zweite Abschnitt (22, 24; 64, 66; 104, 106) etwa parallel zueinander verlaufen und der dritte Abschnitt (26; 68; 108) schräg oder quer zu dem ersten und zweiten Abschnitt verläuft.

3. Bildaufnehmermodul nach Anspruch 1, **dadurch gekennzeichnet, daß** der dritte Abschnitt (154) etwa V-förmig ausgebildet ist.

4. Bildaufnehmermodul nach Anspruch 3, **dadurch gekennzeichnet, daß** zwei Schenkel (153, 155) des V-förmigen dritten Abschnitts (154) in geradliniger Verlängerung des ersten bzw. zweiten Abschnitts (150, 152) verlaufen.

5. Bildaufnehmermodul nach Anspruch 3, **dadurch gekennzeichnet, daß** der erste und der zweite Abschnitt (150, 152) etwa parallel zueinander verlaufen.

6. Bildaufnehmermodul nach Anspruch 1, **dadurch gekennzeichnet, daß** der dritte Abschnitt nach außen oder nach innen gewölbt ist.

7. Bildaufnehmermodul nach einem der Ansprüche 1 bis 6, **dadurch gekennzeichnet, daß** das zumindest eine Kabel (44) auf einer innenliegenden Seite der Leiterplatte (20; 62; 102; 122) kontaktiert ist.

8. Bildaufnehmermodul nach einem der Ansprüche 1 bis 6, insbesondere nach einem der Ansprüche 3 bis 5, **dadurch gekennzeichnet, daß** das zumindest eine Kabel (44) auf einer außenliegenden Seite der Leiterplatte (148), insbesondere auf der Außenseite des V-förmigen dritten Abschnitts (154), kontaktiert ist.

9. Bildaufnehmermodul nach einem der Ansprüche 1 bis 8, **dadurch gekennzeichnet, daß** der dritte Abschnitt (26; 68; 108) zumindest einen Durchgang (52; 76, 78; 116) für das zumindest eine von der Leiterplatte (20; 62; 102; 122) wegführende Kabel (44) aufweist.

10. Bildaufnehmermodul nach Anspruch 9, **dadurch gekennzeichnet, daß** der zumindest eine Durchgang (76, 78) als randseitige Aussparung im dritten Abschnitt (68) ausgebildet ist.

11. Bildaufnehmermodul nach Anspruch 9 oder 10, **dadurch gekennzeichnet, daß** der zumindest eine Durchgang (52; 116) als etwa mittige Öffnung im dritten Abschnitt (26; 108) ausgebildet ist.

12. Bildaufnehmermodul nach einem der Ansprüche 1 bis 11, **dadurch gekennzeichnet, daß** der durch den ersten, zweiten und dritten Abschnitt (22-26; 64-68; 104-108; 150-154) aufgespannte Innenbereich der Leiterplatte (20; 62; 102; 122) mit einer elektrisch nichtleitenden Füllmasse ausgefüllt ist.

13. Bildaufnehmermodul nach einem der Ansprüche 1 bis 12, **dadurch gekennzeichnet, daß** die Leiterplatte (20; 62; 102; 122; 148) einen vierten Abschnitt (32; 70; 110; 156) aufweist, der dem dritten Abschnitt (26; 68; 108; 154) gegenüberliegend angeordnet ist, und auf dessen Außenseite der Bildsensor (12) zu liegen kommt.

14. Bildaufnehmermodul nach Anspruch 13, **dadurch gekennzeichnet, daß** der vierte Abschnitt (32; 70; 156) zumindest ein elektrisches Bauteil und/oder zumindest eine elektrische Leiterbahn aufweist.

15. Bildaufnehmermodul nach einem der Ansprüche 1 bis 14, **dadurch gekennzeichnet, daß** die Leiterplatte (62; 122) zusätzlich zu dem ersten Abschnitt (22; 64) und dem zweiten Abschnitt (24; 66) einen weiteren Abschnitt (72; 126) oder zwei weitere Abschnitte (72, 74; 126, 128) aufweist, der oder die an einer Längsseite der Leiterplatte (62; 122) angeordnet ist/sind.

16. Bildaufnehmermodul nach einem der Ansprüche 1 bis 15, **dadurch gekennzeichnet, daß** die Leiterplatte (20; 62; 102; 122; 148) auf ihrer außenliegenden Seite Vertiefungen zur Kontaktierung des Bildsensors aufweist.

17. Bildaufnehmermodul nach einem der Ansprüche 1 bis 16, **dadurch gekennzeichnet, daß** der dritte Abschnitt (108; 154) der Leiterplatte (102; 148) zumindest einen Kontakt zum Kontaktieren des von der Leiterplatte (102; 148) wegführenden Kabels (44) aufweist.

18. Bildaufnehmermodul nach einem der Ansprüche 1 bis 17, **dadurch gekennzeichnet, daß** der dritte Abschnitt (26; 68; 108; 154) der Leiterplatte (20; 62; 102; 122; 148) zumindest eine elektrische Leiterbahn zum elektrischen Verbinden des ersten Abschnitts (22; 64; 104; 150) und des zweiten Abschnitts (24; 66; 106; 152) aufweist.

19. Bildaufnehmermodul nach einem der Ansprüche 1 bis 18, **dadurch gekennzeichnet, daß** die Leiterplatte (20; 62; 102; 122; 148) aus einem zumindest den ersten, zweiten und dritten Abschnitt (22-26; 64-68; 104-108; 150-154) einstückig aufweisenden planaren Plattenrohling (54; 80; 112; 130) gefaltet ist.

20. Bildaufnehmermodul nach Anspruch 19, **dadurch gekennzeichnet, daß** bei dem Plattenrohling (80) der erste Abschnitt (64) und der zweite Abschnitt (66) über einen weiteren Abschnitt (74) flexibel verbunden, jedoch beabstandet auf gleicher Höhe angeordnet sind, daß der erste Abschnitt (64) und der zweite Abschnitt (66) zum Kontaktieren jeweils zumindest eines Kabels (44) dienen, und daß der dritte Abschnitt (68) an einer Stirnseite des weiteren Abschnitts (74) flexibel mit diesem verbunden ist.

21. Verfahren zum Zusammenbauen eines Bildaufnehmermoduls, bei dem ein elektronischer Bildsensor (12) mit einer einstückigen Leiterplatte (20; 62; 102; 122; 148) elektrisch kontaktiert wird und die Leiterplatte (20; 62; 102; 122; 148) mit zumindest einem von der Leiterplatte (20; 62; 102; 122; 148) wegführenden Kabel (44) elektrisch kontaktiert wird, **dadurch gekennzeichnet, daß** zunächst die Leiterplatte (20; 62; 102; 122; 148) als planarer Plattenrohling (54; 80; 112; 130) vorliegt, der zumindest drei Abschnitte (22-26; 64-68; 104-108; 150-154) aufweist, die entlang flexibler Verbindungsabschnitte (28, 30, 34; 82-90) faltbar sind, daß das zumindest eine Kabel (44) mit dem Plattenrohling (54; 80; 112; 130) kontaktiert wird, daß anschließend der Plattenrohling (54; 80; 112; 130) derart gefaltet wird, daß ein dritter Abschnitt (26; 68; 108; 154) zwischen einem ersten Abschnitt (22; 64; 104; 150) und einem zweiten Abschnitt (24; 66; 106; 152) angeordnet ist, und daß anschließend der Bildsensor (12) an einem dem dritten Abschnitt (26; 68; 108; 154) gegenüberliegenden Ende der Leiterplatte (20; 62; 102; 122; 148) angeordnet und mit dieser kontaktiert wird, und daß der dritte Abschnitt (26; 68; 108; 154) zur Zugentlastung des zumindest einen Kabels (44) verwendet wird.

22. Verfahren nach Anspruch 21, **dadurch gekennzeichnet, daß** der Innenbereich der Leiterplatte (20; 62; 102; 122; 148) nach dem Kontaktieren des zumindest einen Kabels (44) mit einer aushärtenden elektrisch nichtleitenden Füllmasse aufgefüllt wird.

## Claims

1. Image pick-up module, especially for an endoscope, comprising an electronic image sensor (12), a single-piece circuit board (20; 62; 102; 122; 148) which is electrically bonded to the image sensor (12) and to which is further electrically bonded at least one cable (44) leading away from the circuit board (20; 62; 102; 122; 148), the circuit board (20; 62; 102; 122; 148) having at least three sections (22, 24, 26; 64, 66, 68; 104, 106, 108; 150, 152, 154), with a first section (22; 64; 104; 150) and a second section (24; 66; 106; 152) extending in spaced relation one to the other and obliquely or crosswise to the image sensor (12) and a third section (26; 68; 108; 154) being arranged between the first and the second sections (22, 24; 64, 66; 104, 106), wherein the image sensor (12) is arranged on one end of the circuit board (20; 62; 102; 122; 148) opposite the third section (26; 68; 108; 154), **characterized in that** the third section (26; 68; 108; 154) serves for the strain relief of the at least one cable (44).

2. The image pick-up module of Claim 1, **characterized in that** the first and the second sections (22, 24; 64, 66; 104, 106) extend substantially in parallel one to the other, and the third section (26; 68; 208) extends obliquely or crosswise to the first and second sections.

3. The image pick-up module of Claim 1, **characterized in that** the third section (154) has a substantially V-shaped configuration.

4. The image pick-up module of Claim 3, **characterized in that** two legs (153, 155) of the V-shaped third section (154) extend along a straight-line prolongation of the first and the second sections (150, 152), respectively.

5. The image pick-up module of Claim 3, **characterized in that** the first and the second sections (150, 152) extend substantially in parallel one to the other.

6. The image pick-up module of Claim 1, **characterized in that** the third section is curved in outward or inward direction.

7. The image pick-up module of anyone of Claims 1 through 6, **characterized in that** the at least one cable (44) is bonded to an inner surface of the circuit board (20; 62; 102; 122).

8. The image pick-up module of anyone of Claims 1 through 6, especially of anyone of Claims 3 through 5, **characterized in that** the at least one cable (44) is bonded to an outer surface of the circuit board (148), especially to the outside of the V-shaped third section (154).

9. The image pick-up module of anyone of Claims 1 through 8, **characterized in that** the third section (26; 68; 108) comprises at least one passage (52; 76, 78; 116) for the at least one cable (44) leading away from the circuit board (20; 62; 102; 122).

10. The image pick-up module of Claim 9, **characterized in that** the at least one passage (76, 78) is configured as a marginal recess in the third section (68).

11. The image pick-up module of Claim 9 or Claim 10, **characterized in that** the at least one passage (52; 116) is configured as a substantially central opening in the third section (26; 108).

12. The image pick-up module of anyone of Claims 1 through 11, **characterized in that** the interior of the circuit board (20; 62; 102; 122), defined by the first, second and third sections (22-26; 64-68; 104-108; 150-154), is filled with an electrically non-conductive filling compound.

13. The image pick-up module of anyone of Claims 1 through 12, **characterized in that** the circuit board (20; 62; 102; 122; 148) comprises a forth section (32; 70; 110; 156) arranged opposite the third section (26; 68; 108; 154) and accommodating the image sensor (12) on its outer surface.

14. The image pick-up module of Claim 13, **characterized in that** the forth section (32; 70; 156) comprises at least one electric component and/or at least one electric circuit-board conductor.

15. The image pick-up module of anyone of Claims 1 through 14, **characterized in that** the circuit board (62; 122) comprises, in addition to the first section (22; 64) and the second section (24; 66), one (72; 126) or two (72, 74; 126, 128) additional sections arranged on one longitudinal side of the circuit board (62; 122).

16. The image pick-up module of anyone of Claims 1 through 15, **characterized in that** the circuit board (20; 62; 102; 122; 148) is provided on its outer surface with recesses for bonding of the image sensor.

17. The image pick-up module of anyone of Claims 1 through 16, **characterized in that** the third section (108; 154) of the circuit board (102; 148) comprises at least one contact for bonding the cable (44) leading away from the circuit board (102; 148).

18. The image pick-up module of anyone of Claims 1 through 17, **characterized in that** the third section (26; 68; 108; 154) of the circuit board (20; 62; 102; 122; 148) comprises at least one electric circuit-board conductor for electrically connecting the first section (22; 64; 104; 150) and the second section (24; 66; 106; 152).

19. The image pick-up module of anyone of Claims 1 through 18, **characterized in that** the circuit board (20; 62; 102; 122; 148) is folded from a planar board blank (54; 80; 112; 130) comprising at least the integrally formed first, second and third sections (22-26; 64-68; 104-108; 150-154).

20. The image pick-up module of Claim 19, **characterized in that** the first section (64) and the second section (66) of the board blank (80) are flexibly connected via an additional section (74), but are arranged in spaced relationship at one and the same level, wherein the first section (64) and the second section (66) each serve for bonding at least one cable (44), and wherein the third section (68) is flexibly connected to the additional section (74) on the end face of the additional section (74).

21. Method for assembling an image pick-up module, according to which an electronic image sensor (12) is electrically bonded to a single-piece circuit board (20; 62; 102; 122; 148) and the circuit board (20; 62; 102; 122; 148) is electrically bonded to at least one cable (44) leading away from the circuit board (20; 62; 102; 122; 148), **characterized in that** the circuit board (20; 62; 102; 122; 148) initially has the form of a planar board blank (54; 80; 102; 130) comprising at least three sections (22-26; 64-69; 104-108; 150-154) that can be folded along flexible connecting sections (28, 30, 34; 82-90), wherein the at least one cable (44) is bonded to the board blank (54; 80; 112; 130), wherein the board blank (54; 80; 112; 130) is then folded in such a way that a third section (26; 68; 108; 154) is located between a first section (22; 64; 104) and a second section (24; 66; 106; 152), and wherein the image sensor (12) is bonded to the circuit board (20; 62; 102; 122; 148) at an end of the board opposite the third section (26; 68; 208; 154), and that the third section (26; 68; 108; 154) is used for the strain relief of the at least one cable (44).

22. The method of Claim 21, **characterized in that** after bonding of the at least one cable (44) the interior of the circuit board (20; 62; 102; 122; 148) is filled up with a curing electrically non-conductive filling compound.

## Revendications

1. Module enregistreur d'image, en particulier pour un endoscope, avec un capteur d'image (12) électronique, avec un circuit imprimé (20 ; 62 ; 102 ; 122 ; 148) d'un seul tenant, qui est mis en contact électrique avec le capteur d'image (12), et avec lequel est mis en contact au moins une câble (44) partant du circuit imprimé (20 ; 62 ; 102 ; 122 ; 148), le circuit imprimé (20 ; 62 ; 102 ; 122 ; 148) présentant au moins trois sections (22, 24, 26 ; 64, 66, 68 ; 104, 106, 108 ; 150, 152, 154), dont une première section (22 ; 64 ; 104 ; 150) et une deuxième section (24 ; 66 ; 106 ; 152) s'étendent à distance l'une de l'autre à l'oblique ou transversalement au capteur d'image (12) et une troisième section (26 ; 68 ; 108 ; 154) est placée entre la première et la deuxième section (22, 24 ; 64, 66 ; 104, 106), le capteur d'image (12) étant placé à une extrémité du circuit imprimé (20 ; 62 ; 102 ; 122 ; 148) opposée à la troisième section (26 ; 68 ; 108 ; 154), **caractérisé en ce que** la troisième section (26 ; 68 ; 108 ; 154) sert à décharger en traction le câble (44) au nombre d'au moins un.

2. Module enregistreur d'image selon la revendication 1, **caractérisé en ce que** la première et la deuxième sections (22, 24 ; 64, 66 ; 104, 106) s'étendant approximativement parallèlement l'une à l'autre et la troisième section (26 ; 68 ; 108) s'étend à l'oblique ou transversalement à la première et à la deuxième section.

3. Module enregistreur d'image selon la revendication 1, **caractérisé en ce que** la troisième section (154) est formée approximativement en V.

4. Module enregistreur d'image selon la revendication 3, **caractérisé en ce que** deux branches (153, 155) de la troisième section (154) en forme de V s'étendent dans le prolongement rectiligne de la première, respectivement deuxième section (150, 152).

5. Module enregistreur d'image selon la revendication 3, **caractérisé en ce que** la première et la deuxième sections (150, 152) s'étendent approximativement parallèlement l'une à l'autre.

6. Module enregistreur d'image selon la revendication 1, **caractérisé en ce que** la troisième section est bombée vers l'extérieur ou vers l'intérieur.

7. Module enregistreur d'image selon l'une des revendications 1 à 6, **caractérisé en ce que** le câble (44) au nombre d'au moins un est mis en contact sur un côté intérieur du circuit imprimé (20 ; 62 ; 102 ; 122).

8. Module enregistreur d'image selon l'une des revendications 1 à 6, en particulier selon l'une des revendications 3 à 5, **caractérisé en ce que** le câble (44) au nombre d'au moins un est mis en contact sur un côté extérieur du circuit imprimé (148), en particulier sur le côté extérieur de la troisième section (154) en forme de V.

9. Module enregistreur d'image selon l'une des revendications 1 à 8, **caractérisé en ce que** la troisième section (26 ; 68 ; 108) présente au moins un passage (52 ; 76 ; 78 ; 116) pour le câble (44) au nombre d'au moins un partant du circuit imprimé (20 ; 62 ; 102 ; 122).

10. Module enregistreur d'image selon la revendication 9, **caractérisé en ce que** le passage (76, 78) au nombre d'au moins un est conformé en évidement situé sur le bord dans la troisième section (68).

11. Module enregistreur d'image selon la revendication 9 ou 10, **caractérisé en ce que** le passage (52 ; 116) au nombre d'au moins un est conformé en ouverture approximativement centrale dans la troisième section (26 ; 108).

12. Module enregistreur d'image selon l'une des revendications 1 à 11, **caractérisé en ce que** la zone intérieure du circuit imprimé (20 ; 62 ; 102 ; 122) couverte par les première, deuxième et troisième sections (22-26 ; 64-68 ; 104-108 ; 150-154) est remplie d'une masse de remplissage non conductrice électrique.

13. Module enregistreur d'image selon l'une des revendications 1 à 12, **caractérisé en ce que** le circuit imprimé (26 ; 62 ; 102 ; 122 ; 148) présente une quatrième section (32 ; 70 ; 110 ; 156) qui est placée en vis-à-vis de la troisième section (26 ; 68 ; 108 ; 154) et sur la face extérieure de laquelle vient se placer le capteur d'image (12).

14. Module enregistreur d'image selon la revendication 13, **caractérisé en ce que** la quatrième section (32 ; 70 ; 156) présente au moins un composant électrique et/ou au moins une piste conductrice électrique.

15. Module enregistreur d'image selon l'une des revendications 1 à 14, **caractérisé en ce que** le circuit imprimé (62 ; 122) présente en plus de la première section (22 ; 64) et de la deuxième section (24 ; 66) une autre section (72 ; 126) ou deux autres sections (72, 74 ; 126, 128) qui est/sont placée(s) sur un côté longitudinal du circuit imprimé (62 ; 122).

16. Module enregistreur d'image selon l'une des revendications 1 à 15,
**caractérisé en ce que** le circuit imprimé (20 ; 62 ; 102 ; 122 ; 148) présente sur sa face extérieure des renfoncements pour la mise en contact du capteur d'image.

17. Module enregistreur d'image selon l'une des revendications 1 à 16, **caractérisé en ce que** la troisième section (108 ; 154) du circuit imprimé (102 ; 148) présente au moins un contact pour la mise en contact du câble (44) partant du circuit imprimé (102 ; 148).

18. Module enregistreur d'image selon l'une des revendications 1 à 17, **caractérisé en ce que** la troisième section (26 ; 68 ; 108 ; 154) du circuit imprimé (20 ; 62 ; 102 ; 122 ; 148) présente au moins une piste conductrice pour la connexion électrique de la première section (22 ; 64 ; 104 ; 150) et de la deuxième section (24 ; 66 ; 106 ; 152).

19. Module enregistreur d'image selon l'une des revendications 1 à 18, **caractérisé en ce que** le circuit imprimé (20 ; 62 ; 102 ; 128 ; 148) est plié à partir d'une ébauche plane (54 ; 80 ; 112 ; 130) présentant au moins les première, deuxième et troisième sections (22-26 ; 64-68 ; 104-108 ; 150-154) d'un seul tenant.

20. Module enregistreur d'image selon la revendication 19, **caractérisé en ce que**, dans l'ébauche plane (80), la première section (64) et la deuxième section (66) sont reliées de manière souple par l'intermédiaire d'une autre section (74), mais sont placées à distance à même hauteur, **en ce que** la première section (64) et la deuxième section (66) servent chacune à la mise en contact d'au moins un câble (44), et **en ce que** la troisième section (68) est reliée, sur une face frontale de l'autre section (74), de manière souple à celle-ci.

21. Procédé d'assemblage d'un module enregistreur d'image, dans lequel un capteur électronique d'image (12) est mis en contact électrique avec un circuit imprimé (20 ; 62 ; 102 ; 122 ; 148) d'un seul tenant et le circuit imprimé (20 ; 62 ; 102 ; 122 ; 148) est mis en contact électrique avec au moins une câble (44) partant du circuit imprimé (20 ; 62 ; 102 ; 122 ; 148), **caractérisé en ce que** le circuit imprimé (20 ; 62 ; 102 ; 122 ; 148) est d'abord présent sous la forme d'une ébauche plane (54 ; 80 ; 112 ; 130) qui présente au moins trois sections (22-26 ; 64-68 ; 104-108 ; 150-154) qui sont pliables le long de sections de liaison flexibles (28, 30, 34 ; 82-90), **en ce que** le câble (44) au nombre d'au moins un est mis en contact avec l'ébauche plane (54 ; 80 ; 112 ; 130), **en ce que** l'ébauche plane (54 ; 80 ; 112 ; 130) est ensuite pliée de telle manière qu'une troisième section (26 ; 68 ; 108 ; 154) se trouve entre une première section (22 ; 64 ; 104 ; 150) et une deuxième section (24 ; 66 ; 106 ; 152), et **en ce que** le capteur d'image (12) est ensuite placé à une extrémité du circuit imprimé (20 ; 62 ; 102 ; 122 ; 148) opposée à la troisième section (26 ; 68 ; 108 ; 154) et est mis en contact avec celui-ci, et **en ce que** la troisième section (26 ; 68 ; 108 ; 154) est employée pour soulager la traction du câble (44) au nombre d'au moins un.

22. Procédé selon la revendication 21, **caractérisé en ce que** la partie intérieure du circuit imprimé (20 ; 62 ; 102 ; 122 ; 148) est remplie d'une masse de remplissage durcissante non conductrice électrique après la mise en contact du câble (44) au nombre d'au moins un.
